# EUROPEAN PATENT SPECIFICATION

(11) **EP 4 506 338 B1**
(45) Date of publication and mention of the grant of the patent: **18.02.2026**
(21) Application number: 24170811.4
(22) Date of filing: 17.04.2024
(51) Int. Cl.: C07D 207/28

(54) **METHOD FOR PREPARING 2-HYDROXY-5-OXOPROLINE**
VERFAHREN ZUR HERSTELLUNG VON 2-HYDROXY-5-OXOPROLIN
PROCÉDÉ DE PRÉPARATION DE 2-HYDROXY-5-OXOPROLINE

(30) Priority: 07.08.2023 US 202318231051; 15.02.2024 WO PCT/US2024/016056
(43) Date of publication of application: 12.02.2025
(73) Proprietor: Millennium Enterprises, Inc., Marietta, GA 30066 (US)
(72) Inventor: MARTINEZ, Rodolfo, Santa Fe, New Mexico (US); MARTINEZ, Nathan, Santa Fe, New Mexico (US); BLASKOVIC, Robert, Marietta, Georgia (US); GLASS, David Rembert, Santa Fe, New Mexico (US); UNKEFER, Clifford J, Los Alamos, New Mexico (US)
(74) Representative: J A Kemp LLP

(56) References cited:
- US-B1- 6 288 240
- DUFFY T E ET AL: "Acid-catalyzed hydrolysis of @a-ketoglutaramic acid - A proposed mechanism involving decarboxylation and amide hydrolysis of the @c-lactam, 2-pyrrolidone-5-hydroxy-5-carboxylic acid", BIOORGANIC CHEMISTRY, ACADEMIC PRESS INC., NEW YORK, NY, US, vol. 5, no. 4, 1 December 1976 (1976-12-01), pages 351 - 366, XP024022382, ISSN: 0045-2068, [retrieved on 19761201], DOI: 10.1016/0045-2068(76)90020-1
- TOMIKO KUHARA ET AL: "Urinary 2-hydroxy-5-oxoproline, the lactam form of � -ketoglutaramate, is markedly increased in urea cycle disorders", ANALYTICAL AND BIOANALYTICAL CHEMISTRY, SPRINGER, BERLIN, DE, vol. 400, no. 7, 6 February 2011 (2011-02-06), pages 1843 - 1851, XP019905723, ISSN: 1618-2650, DOI: 10.1007/S00216-011-4688-X
- COOPER ARTHUR J. ET AL: "The Glutamine Transaminase-[omega]-Amidase Pathwa", vol. 4, no. 3, 1 January 1977 (1977-01-01), pages 281 - 303, XP093207399, ISSN: 0045-6411, Retrieved from the Internet <URL:https://dx.doi.org/10.3109/10409237709102560> DOI: 10.3109/10409237709102560

## Description

### TECHNICAL FIELD

Embodiments of the present invention are generally related to 2-hydroxy-5-oxoproline. More particularly, embodiments are related to methods for preparing a 2-hydroxy-5-oxoproline salt. Embodiments are also related to the preparation of 2-hydroxy-5-oxoproline salt by dissolving Sodium 2-pyrrolidone-5-carboxylate (sodium pyroglutamate) in aqueous solution and reacting a molar excess of ozone with the aqueous solution of sodium 2-pyrrolidone-5-carboxylate at a temperature between 20°C and 100°C such that sodium 2-hydroxy-5-oxoproline is formed.

### BACKGROUND

The metabolite 2-hydroxy-5-oxoproline (2-oxoglutaramate) was discovered several decades ago in animal livers and kidneys and investigated in these tissues. A limited understanding of its function in animals was developed from these studies, in cells this material appears to be made by the physiologically irreversible transamination of glutamine. The compound appears to play a role in the regulation of tissue glutamine levels and in ammonia genesis, that is, the amide nitrogen in glutamine is a major Source of urinary ammonia. See, e.g., "The Glutamine Transaminase-co-Amidase Pathway" by Arthur J. L. Cooper and Alton Meister, CRC Critical Reviews in Biochemistry, pages 281-303, January 1977, and "Occurrence Of Glutamine-2-Oxoacid Transaminase Activity In The Blue Green Alga ANABAENACYLINDRICA" by Masayuki Ohmori et al., J. Gen Appl. Microbiol. 31, 171 (1985).

More recently, 2-hydroxy-5-oxoproline has been found to have significant effect on plant growth. See, e.g., "Use Of Prolines For Improving Growth And Other Properties Of Plants And Algae' by Pat J. Unkefer, Thomas J. Knight, and Rodolfo A. Martinez, U.S. Patent and Trademark Office Ser. No. 09/493,039, filed on Jan. 27, 2000, where the inventors describe the use of the chemical class of compounds known as prolines for improving plant properties and performance.

In the past, 2-hydroxy-5-oxoproline has been synthesized using enzymatic procedures which are slow and difficult to control. See, e.g., A. L. Cooper and Alton Meister, Supra.

The reaction of Fremy's Salt with glutamic acid to yield (21%) an a-ketoacid of glutamic acid is described in "Oxidation of a-Amino Acids and a-Hydroxy Acids By Fremy's Salt. A Model For Oxidases?" by Angel Garcia-Raso, J. Org. Chem. 51,4285 (1986). More particularly, L-glutamic acid was used as the starting material.

The quantitative chemical synthesis for this 2-hydroxy-5-oxoproline has been performed in a single step reaction of Fremy's Salt with either glutamine or 2-pyrrolidone-5-carboxylic acid, as was described by one of the present inventors (Martinez) in U.S. Patent No. 6,288,240, entitled "preparation of 2-hydroxy-5-oxoproline and analogs thereof", which was granted September 11, 2001. A product was produced in near quantitative yield. However, this process produced a waste stream that prevented its use for the commercial production of 2-hydroxy-5-oxoproline.

What is needed is a process that does not produce any waste and can be used as the commercial product when produced.

### BRIEF SUMMARY

The following summary is provided to facilitate an understanding of some of the features of the disclosed embodiments and is not intended to be a full description. A full appreciation of the various aspects of the embodiments disclosed herein can be gained by taking the specification, claims, drawings, and abstract as a whole.

The invention provides a method for preparing a 2-hydroxy-5-oxoproline salt which comprises the steps of:
(a) providing an aqueous solution of a 2-pyrrolidone-5-carboxylate salt; and
(b) oxidizing the 2-pyrrolidone-5-carboxylate salt in solution using an oxidizing agent such that the 2-hydroxy-5-oxoproline salt is formed,
   wherein the oxidizing agent comprises ozone and/or hydrogen peroxide, and wherein the oxidation step occurs at a temperature between 20°C and 100°C.

In accordance with a feature of the embodiments, a method is provided for preparing 2-hydroxy-5-oxoproline salt that includes the steps of: dissolving Sodium 2-pyrrolidone-5-carboxylate in aqueous solution and reacting a molar excess of ozone with the aqueous solution of sodium 2-pyrrolidone-5-carboxylate at a temperature between 20°C and 50°C such that sodium 2-hydroxy-5-oxoproline salt is formed.

In accordance with another feature of the embodiments, an aqueous solution of hydrogen peroxide can be reacted with the said solution of sodium 2-pyrrolidone-5-carboxylate at a temperature between 20°C and 100°C.

In accordance with yet another feature of the embodiments, a method for preparing 2-hydroxy-5-oxoproline can further include the step of adjusting the pH of an aqueous solution of 2-pyrrolidone-5-carboxylic acid to pH7 with alkali metal hydroxides (i.e., sodium hydroxide, potassium hydroxide, calcium hydroxide, etc.).

In accordance with another feature of the embodiments, a method for preparing 2-hydroxy-5-oxoproline can further include the steps of adjusting the pH of an aqueous solution of 2-pyrrolidone-5-carboxylic acid to pH7 with alkali metal carbonates or bicarbonates (i.e., sodium carbonate, potassium carbonate, sodium bicarbonate, potassium bicarbonate, etc.).

These and other aspects of the embodiments will become more apparent in light of the detailed specification and drawings that follow.

### BRIEF DESCRIPTION OF THE DRAWINGS

The accompanying figures, in which like reference numerals refer to identical or functionally similar elements throughout the separate views and which are incorporated in and form a part of the specification, further illustrate the present invention and, together with the detailed description of the invention, serve to explain the principles of the present invention.
FIG. 1 illustrates a flow diagram of a method for preparing 2-hydroxy-5-oxoproline, in accordance with the embodiments;
FIG. 2 illustrates a diagram of the reaction of pyroglutamic acid with alkali bases;
FIG. 3 illustrates a diagram of a complete reaction sequence which can be done in a single vessel; the 2-hydroxy-5-oxoproline salt that results from the method described herein, in accordance with the embodiments and
FIG. 4 illustrates a diagram of a complete reaction sequence that can be done in a single vessel, in accordance with the embodiments.

### DETAILED DESCRIPTION

The particular values and configurations discussed in these non-limiting examples can be varied and are cited merely to illustrate one or more embodiments and are not intended to limit the scope thereof.

Subject matter will now be described more fully hereinafter with reference to the accompanying drawings, which form a part hereof, and which show, by way of illustration, specific example embodiments. Subject matter may, however, be embodied in a variety of different forms and, therefore, covered or claimed subject matter is intended to be construed as not being limited to any example embodiments set forth herein; example embodiments are provided merely to be illustrative. Among other issues, subject matter may be embodied as methods, devices, components, or systems. Accordingly, embodiments may, for example, take the form of hardware, software, firmware, or a combination thereof. The following detailed description is, therefore, not intended to be interpreted in a limiting sense.

The invention relates to a method for preparing a 2-hydroxy-5-oxoproline salt (also referred to herein as a 4-hydroxypyroglutamate salt). Preferably, the salt is an alkali metal salt or alkaline earth metal salt, and is typically an alkali metal salt. Preferably, the salt is a sodium, potassium or calcium salt, and more preferably is a sodium or potassium salt. Most preferably, the salt is a sodium salt (i.e. the method produces 2-hydroxy-5-oxoproline sodium).

The method comprises providing an aqueous solution of a 2-pyrrolidone-5-carboxylate salt (also referred to herein as a pyroglutamate salt). The counterion of the 2-pyrrolidone-5-carboxylate salt determines the counterion of the resulting 2-hydroxy-5-oxoproline salt. Accordingly, the 2-hydroxy-5-oxoproline salt is preferably an alkali metal salt or alkaline earth metal salt, and is typically an alkali metal salt. Preferably, the salt is a sodium, potassium or calcium salt, and more preferably is a sodium or potassium salt. Most preferably, the salt is a sodium salt (i.e. the method uses sodium 2-pyrrolidone-5-carboxylate).

The step of providing an aqueous solution of a 2-pyrrolidone-5-carboxylate salt may comprise dissolving a solid 2-pyrrolidone-5-carboxylate salt in an aqueous solvent to provide the aqueous solution. Alternatively, the 2-pyrrolidone-5-carboxylate salt may be produced directly in aqueous solution (i.e., not isolated and subsequently dissolved), as a result of the reaction of an aqueous solution of 2-pyrrolidone-5-carboxylic acid with a neutralizing agent as discussed herein.

According to the invention, the 2-pyrrolidone-5-carboxylate salt in solution is oxidized to the 2-hydroxy-5-oxoproline salt using an oxidizing agent, wherein the oxidizing agent comprises ozone and/or hydrogen peroxide. Preferably, the oxidizing agent comprises ozone. More preferably, the oxidizing agent comprises a molar excess of ozone. As used herein, a molar excess of an oxidizing agent is expressed relative to the number of moles of the 2-pyrrolidone-5-carboxylate salt. The number of moles of ozone may be more than one, two, three, four, five or ten times the number of moles of the 2-pyrrolidone-5-carboxylate salt.

The oxidizing agent may comprise hydrogen peroxide. Hydrogen peroxide is typically provided as an aqueous solution. When the oxidizing agent comprises hydrogen peroxide, an aqueous solution of hydrogen peroxide may be added to the aqueous solution of the 2-pyrrolidone-5-carboxylate salt. Alternatively, the 2-pyrrolidone-5-carboxylate salt may be directly dissolved in an aqueous solvent comprising hydrogen peroxide, to provide the aqueous solution. The oxidizing agent may comprise a molar excess of hydrogen peroxide. The number of moles of hydrogen peroxide may be more than one, two, three, four, five or ten times the number of moles of the 2-pyrrolidone-5-carboxylate salt.

The oxidizing agent may comprise ozone and hydrogen peroxide. The ozone and hydrogen peroxide may each be used in molar excess, as outlined for each oxidizing agent above. In this embodiment, the ozone and hydrogen peroxide may be added to the solution of the 2-pyrrolidone-5-carboxylate salt simultaneously or sequentially. Alternatively, the 2-pyrrolidone-5-carboxylate salt may be directly dissolved in an aqueous solvent comprising hydrogen peroxide, to provide the aqueous solution, and then ozone may be subsequently added. Oxidation at the specified temperature may occur after the addition of each oxidizing agent separately, or after both oxidizing agents have been added. For example, the method may comprise dissolving the 2-pyrrolidone-5-carboxylate salt in an aqueous solvent comprising hydrogen peroxide, performing an oxidation step at a temperature between 20°C and 100°C, then adding ozone and further oxidizing at a temperature between 20°C and 50°C.

The oxidation step occurs at a temperature between 20°C and 100°C, for example between 20°C and 90°C, or between 20°C and 80°C, or between 20°C and 70°C, or between 20°C and 60°C, or between 20°C and 50°C, or between 20°C and 40°C, or between 20°C and 30°C. Preferably, the oxidation step occurs at a temperature between 20°C and 50°C, or between 20°C and 40°C, or between 20°C and 30°C. Most preferably, the oxidation step occurs between 20°C and 30°C. In one embodiment, the oxidation step occurs at ambient or room temperature (such as about 25°C).

The oxidation step occurs at a temperature of at least 20°C, for example at least 25°C, at least 30°C, at least 35°C, at least 40°C, at least 45°C, or at least 50°C.

The oxidation step occurs at a temperature of no more than 100°C, for example no more than 90°C, no more than 80°C, no more than 70°C, no more than 60°C, no more than 50°C, no more than 40°C, or no more than 30°C.

The preferred temperature range for the oxidation step may depend on the oxidizing agent that is used. For example, when the oxidizing agent is ozone and hydrogen peroxide is not used as an oxidizing agent, typically the oxidation step occurs at a temperature of between 20°C and 50°C. Alternatively, when hydrogen peroxide is used an oxidizing agent (either alone or in combination with ozone), higher temperatures of up to 100°C (for example between 20°C and 100°C) may be used.

The oxidation step produces the desired 2-hydroxy-5-oxoproline salt. The salt may precipitate out of solution, or may remain dissolved in aqueous solution. Any dissolved salt may be isolated following the reaction (for example, via precipitation or crystallization).

The 2-pyrrolidone-5-carboxylate salt may be produced by providing an aqueous solution of 2-pyrrolidone-5-carboxylic acid, and reacting this aqueous solution with a neutralizing agent. Preferably, the neutralizing agent is selected from an alkali metal hydroxide, carbonate or bicarbonate, and an alkaline earth metal hydroxide, carbonate or bicarbonate. More preferably, the neutralizing agent is selected from an alkali metal hydroxide, an alkaline earth metal hydroxide, an alkali metal carbonate and an alkali metal bicarbonate. Yet more preferably, the neutralizing agent is selected from sodium hydroxide, potassium hydroxide, calcium hydroxide, sodium carbonate, potassium carbonate, sodium bicarbonate, and potassium bicarbonate. Most preferably, the neutralizing agent is a sodium salt, for example sodium hydroxide, sodium carbonate or sodium bicarbonate. More than one neutralizing agent can be used.

Typically, the neutralizing agent is added to the aqueous solution of 2-pyrrolidone-5-carboxylic acid in sufficient amount such that the resulting pH of the solution is about pH 7 (so that the maximum amount of salt has been formed). The method may therefore comprise comprises adjusting the pH of the aqueous solution of 2-pyrrolidone-5-carboxylic acid to about pH 7 (for example to pH 6 to 8, and preferably pH 7) with the neutralizing agent.

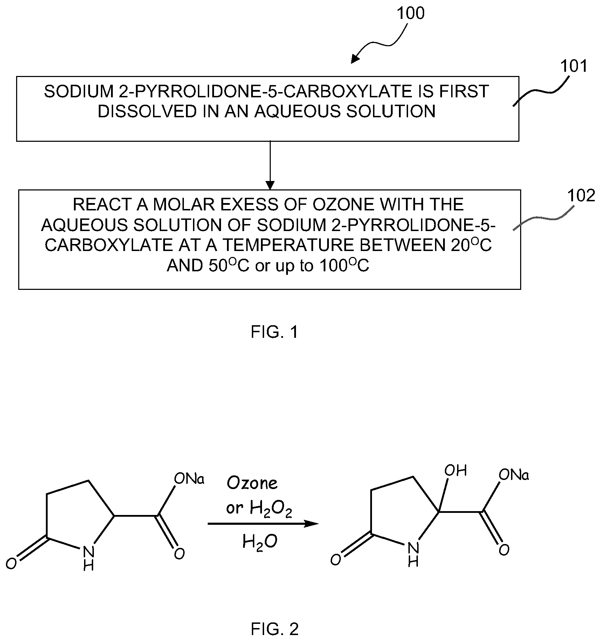

In accordance with a feature of the embodiments, a method is disclosed for preparing 2-hydroxy-5-oxoproline. Referring to FIG. 1 a flow diagram 100 of preparing 2-hydroxy-5-oxoproline is illustrated. Referring to block 101, sodium 2-pyrrolidone-5-carboxylate is first dissolved in an aqueous solution. Then a molar excess of ozone is allowed to react with the aqueous solution of sodium 2-pyrrolidone-5-carboxylate at a temperature between 20°c and 50°c such that sodium 2-hydroxy-5-oxoproline is formed, as show in in Block 102. Referring to FIG. 2, illustrated is a diagram of a reaction of pyroglutamic acid with an alkali bases.

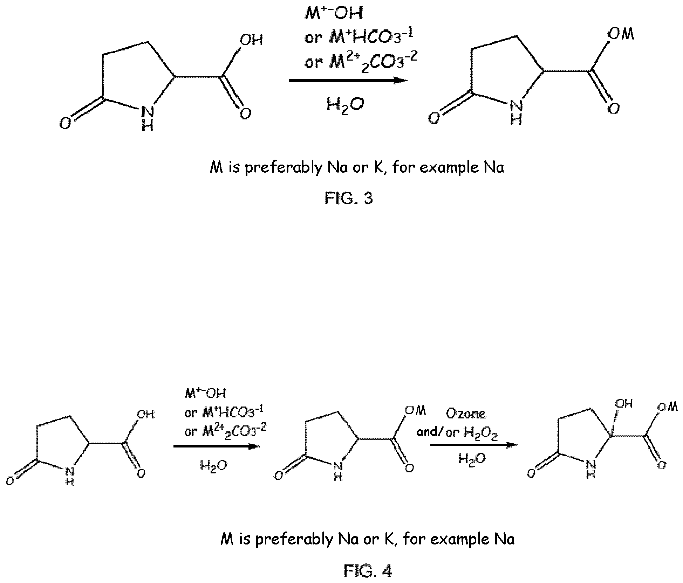

Referring to FIG. 3, illustrated is a diagram of a complete reaction sequence which can be carried out in a single vessel. What is also shown is the 2-hydroxy-5-oxoproline salt that can result from the method described herein, in accordance with the embodiments.

FIG. 4 illustrates another diagram of a complete reaction sequence that can be done in a single vessel, in accordance with the embodiments.

EXAMPLE: sodium L-glutamic acid or sodium L-pyroglutamic acid was reacted with an oxidant such as hydrogen peroxide or ozone. The reaction is allowed to proceed until the 4-hydroxypyroglutamic acid, sodium salt is formed. A typical reaction can be done as follows. Sodium pyroglutamate (20.4 g, 0.13 moles) was dissolved in water (100mL). The homogenous colorless solution (pH = 7) can be placed in an ambient temperature water bath to dissipate any heat produced in the reaction. The desired product can be synthesized using several radical reagents such as hydrogen peroxide (H₂O₂) and Ozone. The NMR and HPLC data are as follow:
NMR Data:
4-hydroxypyroglutamic acid, sodium salt
   ¹³C NMR (H₂O (unlocked), 75 MHz): δ = 29, 34, 89, 177, 181
HPLC (ICSep ICE-ION-300 Column) (1% H₂SO₄, 0.4 mL/min) r.t. = 20.817 min

In this example ozone was bubbled through the solution at 2L/min for a total of 21 hours monitoring the disappearance of the SM (chemical shift = 61 ppm) and subsequent appearance of the desired product (chemical shift = 89 ppm). After this period nitrogen gas can be passed bubbled in the solution to remove excess ozone. An NMR analysis shows the expected 4-hydroxypyroglutamic acid, sodium salt and small amounts of by-products, succinimide and succinic acid sodium salt.

Another example of a typical scaled reaction is as follows: Sodium pyroglutamate (20.4 g, 0.13 moles) was dissolved in water (100mL). The homogenous colorless solution (pH = 7) was placed in an ambient temperature water bath to dissipate any heat produced in the reaction. The desired product has been synthesized using several radical reagents such as hydrogen peroxide (H₂O₂) and Ozone. In this example ozone was bubbled through the solution at 2L/min for a total of 21 hours monitoring the disappearance of the SM (chemical shift = 61 ppm) and subsequent appearance of the desired product (chemical shift = 89 ppm). After this period nitrogen gas is passed bubbled in the solution to remove excess ozone. An NMR analysis shows that the expected 4-hydroxypyroglutamic acid, sodium salt and small amounts of by-products, succinimide and succinamic acid sodium salt.

It can be appreciated that the reaction can be scaled to multikilogram quantities by using a larger ozone generator and larger reactors.

A typical scaled reaction produced 140 Kg of product in 12-13 days of reaction time. The reaction rate can be increased or decreased by regulating the amount of ozone that is introduced to the reaction. Once the reaction is complete, compressed air is bubbled into the reaction to remove excess ozone. The product is used as produced without further purification.

It will be appreciated that variations of the above-disclosed embodiments and examples and other features and functions, or alternatives thereof, may be desirably combined into many other different systems or applications.

Aspects of the disclosure are set out below:
1. A method for preparing 2-hydroxy-5-oxoproline salt which comprises the steps of:
   (a) dissolving sodium 2-pyrrolidone-5-carboxylate in aqueous solution; and
   (b) reacting a molar excess of ozone with the aqueous solution and sodium 2-pyrrolidone-5-carboxylate at a temperature between 20°C and 50°C such that sodium 2-hydroxy-5-oxoproline is formed.
2. The method for preparing 2-hydroxy-5-oxoproline salt as described in aspect 1, wherein the aqueous solution is hydrogen peroxide and is reacted with the Sodium 2-pyrrolidone-5-carboxylate at a temperature between 20°C and 100°C.
3. The method for preparing 2-hydroxy-5-oxoproline salt as described in aspect 1, further including the step of adjusting the pH of the aqueous solution and 2-pyrrolidone-5-carboxylate to pH7 with alkali metal hydroxides.
4. The method for preparing 2-hydroxy-5-oxoproline salt as described in aspect 3, wherein the alkali metal hydroxides can be selected from a group including: sodium hydroxide, potassium hydroxide, calcium hydroxide.
5. The method for preparing 2-hydroxy-5-oxoproline salt as described in aspect 2, further including the step of adjusting the pH of an aqueous solution and 2-pyrrolidone-5-carboxylate to pH7 with alkali metal hydroxides.
6. The method for preparing 2-hydroxy-5-oxoproline salt as described in aspect 5, wherein the alkali metal hydroxides are selected from a group including: sodium hydroxide, potassium hydroxide, calcium hydroxide.
7. The method for preparing 2-hydroxy-5-oxoproline salt as described in aspect 1, further including the step of adjusting the pH of an aqueous solution and 2-pyrrolidone-5-carboxylate to pH7 with alkali metal carbonates or bicarbonates.
8. The method for preparing 2-hydroxy-5-oxoproline salt as described in aspect 7, wherein the alkali metal carbonates or bicarbonates are selected from a group including: sodium carbonate, potassium carbonate, sodium bicarbonate, potassium bicarbonate.
9. The method for preparing 2-hydroxy-5-oxoproline salt as described in aspect 2, further including the step of adjusting the pH of an aqueous solution and 2-pyrrolidone-5-carboxylate to pH7 with alkali metal carbonates or bicarbonates.
10. The method for preparing 2-hydroxy-5-oxoproline salt as described in aspect 9, wherein the alkali metal carbonates or bicarbonates are selected from a group including: sodium carbonate, potassium carbonate, sodium bicarbonate, potassium bicarbonate.
11. A method for preparing 2-hydroxy-5-oxoproline salt which comprises the steps of:
   (a) dissolving sodium 2-pyrrolidone-5-carboxylate in hydrogen peroxide; and
   (b) reacting a molar excess of ozone with the hydrogen peroxide and sodium 2-pyrrolidone-5-carboxylate at a temperature between 20°C and 100°C such that sodium 2-hydroxy-5-oxoproline is formed.
12. The method for preparing 2-hydroxy-5-oxoproline salt as described in aspect 1 or aspect 11, further including the step of adjusting the pH of the hydrogen peroxide and 2-pyrrolidone-5-carboxylate to pH7 with alkali metal hydroxides.
13. The method for preparing 2-hydroxy-5-oxoproline salt as described in aspect 12, wherein the alkali metal hydroxides can be selected from a group including: sodium hydroxide, potassium hydroxide, calcium hydroxide.
14. The method for preparing 2-hydroxy-5-oxoproline salt as described in aspect 1 or aspect 11, further including the step of adjusting the pH of an aqueous solution and 2-pyrrolidone-5-carboxylate to pH7 with alkali metal carbonates or bicarbonates
15. The method for preparing 2-hydroxy-5-oxoproline salt as described in aspect 14, wherein the alkali metal carbonates or bicarbonates are selected from a group including: sodium carbonate, potassium carbonate, sodium bicarbonate, potassium bicarbonate.
16. A method for preparing 2-hydroxy-5-oxoproline salt which comprises the steps of:
   (a) dissolving sodium 2-pyrrolidone-5-carboxylate in aqueous solution;
   (b) reacting a molar excess of ozone with the aqueous solution and sodium 2-pyrrolidone-5-carboxylate at a temperature between 20°C and 50°C such that sodium 2-hydroxy-5-oxoproline is formed; and
   (c) adjusting the pH of the aqueous solution and 2-pyrrolidone-5-carboxylate to pH7 with at least one of alkali metal hydroxides, alkali metal carbonates or alkali metal bicarbonates.
17. The method for preparing 2-hydroxy-5-oxoproline salt as described in aspect 16, wherein the alkali metal hydroxides can be selected from a group including: sodium hydroxide, potassium hydroxide, calcium hydroxide.
18. The method for preparing 2-hydroxy-5-oxoproline salt as described in aspect 16, wherein the alkali metal carbonates or bicarbonates are selected from a group including: sodium carbonate, potassium carbonate, sodium bicarbonate, potassium bicarbonate.
19. The method for preparing 2-hydroxy-5-oxoproline salt as described in aspect 16, wherein the aqueous solution is hydrogen peroxide and is reacted with the Sodium 2-pyrrolidone-5-carboxylate at a temperature between 20°C and 100°C.
20. The method for preparing 2-hydroxy-5-oxoproline salt as described in aspect 19, wherein the alkali metal hydroxides can be selected from a group including: sodium hydroxide, potassium hydroxide, calcium hydroxide.
21. The method for preparing 2-hydroxy-5-oxoproline salt as described in aspect 19, wherein the alkali metal carbonates or bicarbonates are selected from a group including: sodium carbonate, potassium carbonate, sodium bicarbonate, potassium bicarbonate.

## Claims

1. A method for preparing a 2-hydroxy-5-oxoproline salt which comprises the steps of:
(a) providing an aqueous solution of a 2-pyrrolidone-5-carboxylate salt; and
(b) oxidizing the 2-pyrrolidone-5-carboxylate salt in solution using an oxidizing agent such that the 2-hydroxy-5-oxoproline salt is formed,
wherein the oxidizing agent comprises ozone and/or hydrogen peroxide, and wherein the oxidation step occurs at a temperature between 20°C and 100°C.

2. The method according to claim 1, wherein the oxidizing agent comprises a molar excess of ozone.

3. The method according to claim 1 or claim 2, wherein the oxidation step occurs at a temperature between 20°C and 50°C.

4. The method according to claim 1 or claim 2, wherein the oxidizing agent comprises an aqueous solution of hydrogen peroxide.

5. The method according to any one of the preceding claims, wherein the 2-pyrrolidone-5-carboxylate salt is sodium 2-pyrrolidone-5-carboxylate, and the method produces 2-hydroxy-5-oxoproline sodium.

6. The method according to any one of claims 1 to 4, wherein the method comprises the steps of:
(a) providing an aqueous solution of 2-pyrrolidone-5-carboxylic acid;
(b) reacting the aqueous solution of 2-pyrrolidone-5-carboxylic acid with a neutralizing agent selected from an alkali metal hydroxide, carbonate or bicarbonate, and an alkaline earth metal hydroxide, carbonate or bicarbonate, to give an aqueous solution of a 2-pyrrolidone-5-carboxylate alkali metal salt; and
(c) oxidizing the 2-pyrrolidone-5-carboxylate salt using the oxidizing agent such that the 2-hydroxy-5-oxoproline salt is formed.

7. The method according to claim 6, wherein step (b) comprises adjusting the pH of the aqueous solution of 2-pyrrolidone-5-carboxylic acid to pH 7 with the neutralizing agent.

8. The method according to claim 6 or claim 7, wherein the neutralizing agent is an alkali metal hydroxide or alkaline earth metal hydroxide.

9. The method according to any one of claims 6 to 8, wherein the alkali metal hydroxide or alkaline earth metal hydroxide is selected from sodium hydroxide, potassium hydroxide and calcium hydroxide.

10. The method according to claim 6 or claim 7, wherein the neutralizing agent is an alkali metal carbonate or an alkali metal bicarbonate.

11. The method according to any one of claims 6, 7 or 10, wherein the alkali metal carbonate or alkali metal bicarbonate is selected from sodium carbonate, potassium carbonate, sodium bicarbonate, and potassium bicarbonate.

12. The method according to any one of claims 6 to 11, wherein the neutralizing agent is a sodium salt, the 2-pyrrolidone-5-carboxylate alkali metal salt is sodium 2-pyrrolidone-5-carboxylate, and the method produces 2-hydroxy-5-oxoproline sodium.

## Patentansprüche

1. Verfahren zum Herstellen eines 2-Hydroxy-5-oxoprolinsalzes, das die Schritte umfasst:
(a) Bereitstellen einer wässrigen Lösung eines 2-Pyrrolidon-5-carboxylatsalzes; und
(b) Oxidieren des 2-Pyrrolidon-5-carboxylatsalzes in Lösung unter Verwendung eines Oxidationsmittels, sodass das 2-Hydroxy-5-oxoprolinsalz ausgebildet wird,
wobei das Oxidationsmittel Ozon und/oder Wasserstoffperoxid umfasst und wobei der Oxidationsschritt bei einer Temperatur zwischen 20 °C und 100 °C stattfindet.

2. Verfahren nach Anspruch 1, wobei das Oxidationsmittel einen molaren Überschuss an Ozon umfasst.

3. Verfahren nach Anspruch 1 oder 2, wobei der Oxidationsschritt bei einer Temperatur zwischen 20 °C und 50 °C stattfindet.

4. Verfahren nach Anspruch 1 oder 2, wobei das Oxidationsmittel eine wässrige Lösung von Wasserstoffperoxid umfasst.

5. Verfahren nach einem der vorstehenden Ansprüche, wobei das 2-Pyrrolidon-5-carboxylatsalz Natrium-2-pyrrolidon-5-carboxylat ist und das Verfahren 2-Hydroxy-5-oxoprolinnatrium erzeugt.

6. Verfahren nach einem der Ansprüche 1 bis 4, wobei das Verfahren die Schritte umfasst:
(a) Bereitstellen einer wässrigen Lösung von 2-Pyrrolidon-5-carbonsäure;
(b) Reagieren der wässrigen Lösung von 2-Pyrrolidon-5-carbonsäure mit einem Neutralisationsmittel, ausgewählt aus einem Alkalimetallhydroxid, -carbonat oder -bicarbonat und einem Erdalkalimetallhydroxid, -carbonat oder -bicarbonat, um eine wässrige Lösung eines 2-Pyrrolidon-5-carboxylatalkalimetallsalzes zu ergeben; und
(c) Oxidieren des 2-Pyrrolidon-5-carboxylatsalzes unter Verwendung des Oxidationsmittels, sodass das 2-Hydroxy-5-oxoprolinsalz ausgebildet wird.

7. Verfahren nach Anspruch 6, wobei Schritt (b) das Anpassen des pH-Werts der wässrigen Lösung von 2-Pyrrolidon-5-carbonsäure auf einen pH-Wert 7 mit dem Neutralisationsmittel umfasst.

8. Verfahren nach Anspruch 6 oder 7, wobei das Neutralisationsmittel ein Alkalimetallhydroxid oder Erdalkalimetallhydroxid ist.

9. Verfahren nach einem der Ansprüche 6 bis 8, wobei das Alkalimetallhydroxid oder das Erdalkalimetallhydroxid aus Natriumhydroxid, Kaliumhydroxid und Calciumhydroxid ausgewählt ist.

10. Verfahren nach Anspruch 6 oder 7, wobei das Neutralisationsmittel ein Alkalimetallcarbonat oder ein Alkalimetallbicarbonat ist.

11. Verfahren nach einem der Ansprüche 6, 7 oder 10, wobei das Alkalimetallcarbonat oder Alkalimetallbicarbonat aus Natriumcarbonat, Kaliumcarbonat, Natriumbicarbonat und Kaliumbicarbonat ausgewählt ist.

12. Verfahren nach einem der Ansprüche 6 bis 11, wobei das Neutralisationsmittel ein Natriumsalz ist, das 2-Pyrrolidon-5-carboxylatalkalimetallsalz Natrium-2-pyrrolidon-5-carboxylat ist und das Verfahren Natrium-2-hydroxy-5-oxoprolin erzeugt.

## Revendications

1. Procédé de préparation d'un sel de 2-hydroxy-5-oxoproline qui comprend les étapes consistant à :
(a) fournir une solution aqueuse d'un sel 2-pyrrolidone-5-carboxylate ; et
(b) oxyder le sel 2-pyrrolidone-5-carboxylate en solution à l'aide d'un agent oxydant de telle sorte que le sel de 2-hydroxy-5-oxoproline est formé,
dans lequel l'agent oxydant comprend de l'ozone et/ou du peroxyde d'hydrogène, et dans lequel l'étape d'oxydation a lieu à une température comprise entre 20 °C et 100 °C.

2. Procédé selon la revendication 1, dans lequel l'agent oxydant comprend un excès molaire d'ozone.

3. Procédé selon la revendication 1 ou la revendication 2, dans lequel l'étape d'oxydation a lieu à une température comprise entre 20 °C et 50 °C.

4. Procédé selon la revendication 1 ou la revendication 2, dans lequel l'agent oxydant comprend une solution aqueuse de peroxyde d'hydrogène.

5. Procédé selon l'une quelconque des revendications précédentes, dans lequel le sel 2-pyrrolidone-5-carboxylate est le 2-pyrrolidone-5-carboxylate de sodium, et le procédé produit de la 2-hydroxy-5-oxoproline sodique.

6. Procédé selon l'une quelconque des revendications 1 à 4, dans lequel le procédé comprend les étapes consistant à :
(a) fournir une solution aqueuse d'acide 2-pyrrolidone-5-carboxylique ;
(b) faire réagir la solution aqueuse d'acide 2-pyrrolidone-5-carboxylique avec un agent neutralisant sélectionné parmi un hydroxyde, carbonate ou bicarbonate de métal alcalin, et un hydroxyde, carbonate ou bicarbonate de métal alcalino-terreux, pour donner une solution aqueuse d'un sel 2-pyrrolidone-5-carboxylate de métal alcalin ; et
(c) l'oxydation du sel 2-pyrrolidone-5-carboxylate à l'aide de l'agent oxydant de telle sorte que le sel de 2-hydroxy-5-oxoproline est formé.

7. Procédé selon la revendication 6, dans lequel l'étape (b) comprend l'ajustement du pH de la solution aqueuse d'acide 2-pyrrolidone-5-carboxylique à un pH 7 avec l'agent neutralisant.

8. Procédé selon la revendication 6 ou la revendication 7, dans lequel l'agent neutralisant est un hydroxyde de métal alcalin ou un hydroxyde de métal alcalino-terreux.

9. Procédé selon l'une quelconque des revendications 6 à 8, dans lequel l'hydroxyde de métal alcalin ou l'hydroxyde de métal alcalino-terreux est sélectionné parmi l'hydroxyde de sodium, l'hydroxyde de potassium et l'hydroxyde de calcium.

10. Procédé selon la revendication 6 ou la revendication 7, dans lequel l'agent neutralisant est un carbonate de métal alcalin ou un bicarbonate de métal alcalin.

11. Procédé selon l'une quelconque des revendications 6, 7 ou 10, dans lequel le carbonate de métal alcalin ou le bicarbonate de métal alcalin est sélectionné parmi le carbonate de sodium, le carbonate de potassium, le bicarbonate de sodium, et le bicarbonate de potassium.

12. Procédé selon l'une quelconque des revendications 6 à 11, dans lequel l'agent neutralisant est un sel sodique, le sel 2-pyrrolidone-5-carboxylate de métal alcalin est le 2-pyrrolidone-5-carboxylate de sodium, et le procédé produit de la 2-hydroxy-5-oxoproline sodique.
